# FASCICULE DE BREVET EUROPEEN

(11) **EP 3 658 255 B1**
(45) Date de publication et mention de la délivrance du brevet: **24.07.2024**
(21) Numéro de dépôt: 18743813.0
(22) Date de dépôt: 25.07.2018
(51) Int. Cl.: B01D 53/04, G01N 33/00

(54) **DISPOSITIF DE NETTOYAGE D'UN GAZ POUR ANALYSE EN LIGNE**
VORRICHTUNG ZUR REINIGUNG EINES GASES FÜR ONLINE-ANALYSE
DEVICE FOR CLEANING A GAS FOR ON-LINE ANALYSIS

(30) Priorité: 27.07.2017 FR 1757131
(43) Date de publication de la demande: 03.06.2020
(73) Titulaire: Suez International, 92040 Paris La Défense Cedex (FR)
(72) Inventeur: EPHRAÏM, Augustina, 81000 Albi (FR); PHAM MINH, Doan, 81000 Albi (FR); NZIHOU, Ange, 81000 Albi (FR); SHARROCK, Patrick, 31400 Toulouse (FR); LEBONNOIS, Damien, 75015 Paris (FR)
(74) Mandataire: Fédit-Loriot
(86) Numéro de dépôt international: PCT/EP2018/070193
(87) Numéro de publication internationale: WO 2019/020698

(56) Documents cités:
- EP-A1- 0 014 913
- EP-A1- 2 561 916
- EP-A2- 0 239 744
- EP-A2- 0 291 630
- WO-A1-2015/195087
- FR-A1- 2 778 743
- FR-A1- 2 960 446
- FR-A1- 3 032 035
- US-A1- 2002 048 818
- US-A1- 2004 112 117
- US-A1- 2014 262 837

## Description

L'invention se situe dans le domaine du nettoyage de gaz et concerne un dispositif de nettoyage d'un gaz. L'invention peut être appliquée à tout type de gaz et trouve une application particulièrement intéressante dans le nettoyage de gaz (aussi appelé purification de gaz) pour l'analyse en ligne d'un ou plusieurs composés contenus dans le gaz.

L'analyse en ligne de la concentration de certains composés dans un gaz tel que le gaz de synthèse peut s'avérer difficile quand ce gaz contient des goudrons. C'est le cas notamment du gaz de synthèse issu d'un procédé de pyrolyse ou de gazéification de biomasse, résidus ou de déchets. On entend par goudrons des composés organiques ayant un poids moléculaire supérieur à celui du benzène, tels que des hydrocarbures aromatiques polycycliques, des amines aromatiques ou des composés inorganiques. Les goudrons condensent à des températures inférieures à 400°C. Il faut donc les éliminer du gaz avant d'analyser le contenu en certains composés, tels que HCl (chlorure d'hydrogène) ou H₂S (sulfure d'hydrogène ou hydrogène sulfuré), ou NH₃ (ammoniac) afin d'éviter sa condensation sur les parois et miroirs dans la cellule de l'analyseur. D'autres gaz condensables, tels que l'eau, ainsi que des particules solides doivent aussi être éliminés du gaz avant d'en faire l'analyse.

Dans la suite, nous prendrons l'exemple de l'analyse du chlorure d'hydrogène HCl dans un gaz de synthèse. L'invention peut s'appliquer à un autre composé, comme le sulfure d'hydrogène ou l'ammoniac, et également à tout type de gaz pouvant contenir des goudrons.

Il existe des méthodes pour éliminer les goudrons dans la ligne d'échantillonnage du gaz de synthèse se basant sur l'utilisation de solvants organiques tels que l'acétone ou l'isopropanol. Ces méthodes présentent l'inconvénient de capter le chlorure d'hydrogène HCl dans la ligne d'échantillonnage du gaz. De plus, une accumulation du chlorure d'hydrogène HCl peut avoir lieu dans l'espace vide du barboteur contenant le solvant organique car le chlorure d'hydrogène est plus dense que le gaz de synthèse et le débit de gaz dans la ligne d'échantillonnage est normalement faible.

Il existe aussi des méthodes pour éviter la condensation d'eau dans la ligne d'échantillonnage se basant sur le prélèvement du gaz par une sonde et des moyens d'abaissement de la pression des gaz prélevés dans une canalisation. Ces moyens comprennent une buse de détente disposée dans la sonde et un dispositif d'aspiration. Ces systèmes de l'art antérieur peuvent aussi comprendre des moyens de chauffage pour augmenter la température de la canalisation au-dessus du point de rosée d'eau dans le gaz prélevé. Ces méthodes de l'art antérieur sont limitées aux gaz contenant de l'eau et non des goudrons. Pour éviter la condensation de goudrons dans ce type de système, la canalisation doit être chauffée pour augmenter la température de celle-ci au-dessus du point de rosée des goudrons, soit environ 400°C. Le coût d'une telle ligne est relativement élevé, à la fois en termes de coûts des matériaux pour la canalisation et de chauffage.

Enfin, il existe aussi dans l'art antérieur des méthodes pour enlever les particules solides du gaz prélevé. Ces méthodes utilisent un filtre solide, par exemple en laine de silice fondue désactivée, dans une sonde de prélèvement du gaz. Avec un gaz comprenant des goudrons comme le gaz de synthèse, le risque de condensation sur le filtre dans la sonde due aux goudrons existe, ce qui peut bloquer le filtre.

Le document FR 2960446 concerne un média filtrant dit média piégeur comprenant un composé piégeur de composés organovolatils présents dans l'air, le composé piégeur comprenant un groupement NH comme par exemple l'acétoacétamide.

Le document US 2014/262837 concerne un dispositif pour détecter un ou plusieurs composés gazeux cibles, tels qu'un composé organique volatil, tel que le formaldéhyde, comprenant une chambre comprenant une entrée de gaz et une sortie de gaz, dans lequel la chambre est capable d'absorber un ou plusieurs non-composés gazeux cibles. Lorsque le dispositif est en cours d'utilisation, la sortie de gaz est en communication fluidique avec un détecteur capable de détecter la quantité ou la concentration du ou des composés gazeux cibles.

Le document EP 0291630 concerne un processus et appareil dans lequel le gaz à analyser est prétraité en le libérant, notamment de toute teneur en vapeur d'eau. A cette fin, le gaz est d'abord refroidi dans un refroidisseur de gaz, afin de précipiter la vapeur d'eau présente sous forme de condensat, puis passé à travers un filtre aérosol, afin d'éliminer les aérosols formés lors du refroidissement, et enfin libéré de la vapeur d'eau résiduelle dans un sécheur à perméation.

Le document EP0014913A1 concerne un analyseur de dioxyde de soufre qui mesure la teneur en dioxyde de soufre par fluorescence de molécules de dioxyde de soufre lorsqu'elles sont éclairées par une source de lumière ultraviolette. Il est décrit un convertisseur contenant du pentoxyde de vanadium pour l'élimination du polynucléaire les hydrocarbures aromatiques qui produisent des interférences lorsque le dioxyde de soufre est mesuré par la méthodologie fluorescente.

Le document EP 2561916 A1 concerne un procédé d'élimination de naphtalène d'un gaz utilisant un matériau adsorbant solide comprenant du benzène pour l'élimination. La suppression peut être appliquée au flux principal de gaz de synthèse brut et/ou flux latéral d'échappement de dioxyde de carbone.

Le document US 2004/112117 A1 concerne un procédé et un appareil comprenant un filtre pour retirer un gaz non traceur d'un système de détection et une unité de commande pour détecter la présence d'un gaz traceur à faible concentration dans l'atmosphère.

Le document EP 0239744 A2 concerne un procédé pour maintenir propres les tubes de mesure d'appareils de mesure d'émissions d'installations d'épuration de fumées.

En outre, WO 2015/195087 A1 et US 2002/048818 A1 divulguent des ensembles d'analyse en ligne pour déterminer la teneur en NH₃, respectivement en HCl.

L'invention vise à pallier tout ou partie des problèmes cités plus haut en proposant un dispositif d'élimination de goudrons d'un gaz de type gaz de synthèse issu d'un procédé de pyrolyse ou de gazéification de biomasse, résidus ou de déchets, apte à éliminer l'eau et les particules solides éventuellement présentes dans le gaz, adapté au suivi en ligne de la concentration de certains composés contenus dans le gaz.

A cet effet, la description concerne un dispositif de nettoyage d'un gaz comprenant une pluralité de composés, un premier composé parmi la pluralité de composés étant du goudron, et un composé cible comprenant :
un récipient avec une entrée et une sortie, le gaz circulant à travers le récipient depuis l'entrée vers la sortie,
un premier filtre positionné entre l'entrée et la sortie du récipient, à travers lequel le gaz circule, destiné à filtrer le goudron,
et le premier filtre comprenant un matériau absorbant du goudron, le premier filtre ne captant pas ledit composé cible, et le goudron se trouvant sous forme gazeuse, liquide et/ou solide. L'effet technique est de permettre d'absorber le goudron, et donc de l'enlever du gaz, sans pour autant capter les composés que l'on souhaite laisser dans le gaz. Le composé cible reste donc dans le gaz.

Le goudron, en fonction de la température de l'air à analyser, se trouve non seulement sous forme gazeuse, mais également sous forme liquide et/ou solide. Typiquement, le gaz de synthèse se trouve à une température comprise entre 100°C et 1000°C, notamment entre 100°C et 800°C, de préférence entre 100°c et 400°C. Généralement, le gaz à analyser est à une température de 150°C à l'entrée du dispositif de nettoyage. Lorsque le gaz à analyser se trouve à une température inférieure à 400°C, il contient du goudron sous forme de gaz mais également sous forme condensée, c'està-dire sous forme solide et/ou liquide. En particulier, le goudron sous forme de gaz peut capter des poussières solides pour former des particules de goudron solide. Ainsi, selon un mode de réalisation particulier de la description, le goudron dans le gaz à analyser est sous forme gazeuse et solide.

Avantageusement, le matériau absorbant est un matériau naturel, préférentiellement du coton. Le coton est un matériau particulièrement absorbant de goudron.

Selon un autre mode de réalisation, le matériau absorbant est un matériau synthétique, préférentiellement du nylon. Le nylon est un matériau particulièrement absorbant de goudron.

Avantageusement, un deuxième composé parmi la pluralité de composés étant de l'eau, le dispositif peut comprendre un second filtre positionné entre l'entrée et la sortie du récipient, en amont ou en aval du premier filtre, à travers lequel le gaz circule et destiné à enlever l'eau du gaz, et le second filtre comprend un matériau adsorbant de l'eau. Le second filtre ne capte pas ledit composé cible. L'effet technique est de permettre d'enlever l'eau du gaz et de laisser passer les composés que l'on souhaite laisser dans le gaz.

Avantageusement, un troisième composé parmi la pluralité de composés étant un gaz acide, le matériau adsorbant de l'eau comprend une surface de particule neutre ou acide. L'effet technique qui en découle est d'éliminer l'humidité du gaz sans avoir une réaction chimique entre certains composés (par exemple HCl) et le matériau adsorbant de l'eau.

Avantageusement, le matériau adsorbant est une zéolithe. L'effet technique est d'avoir un matériau adsorbant avec des porosités de la structure toutes de la même taille. L'avantage qui en résulte est que la zéolithe adsorbe l'eau et laisse passer le ou les composés souhaités comme le HCl.

Avantageusement, le récipient est un tube comprenant deux extrémités, une première parmi les deux extrémités étant l'entrée et une seconde parmi les deux extrémités étant la sortie. L'avantage est d'éviter la stagnation du gaz dans le récipient.

Selon un mode de réalisation, le tube a une section sensiblement constante entre l'entrée et la sortie. Il en résulte une vitesse d'écoulement du gaz constante dans le récipient.

Selon un autre mode de réalisation, le tube a une section non constante entre l'entrée et la sortie. L'effet technique est d'obtenir une variation de la vitesse d'écoulement du gaz à travers le récipient.

Selon un autre mode de réalisation, le dispositif selon la description peut comprendre un dispositif de chauffage du gaz positionné en amont de l'entrée du récipient. L'effet technique est de chauffer le gaz et le second filtre. Il en résulte un maintien de la température du second filtre au-dessus du point de rosée de l'eau, ce qui évite le phénomène de condensation de l'eau.

Selon un autre mode de réalisation, le dispositif selon la description peut comprendre un dispositif de refroidissement du premier filtre. L'effet technique est d'augmenter la condensation des goudrons et donc l'élimination des goudrons plus efficace. Il en résulte une augmentation du taux de filtrage des goudrons. Un autre effet technique est d'empêcher certains composés du gaz comme le HCl de réagir avec les matériaux.

Avantageusement, le dispositif selon la description comprend un dispositif d'aspiration du gaz positionné en aval de la sortie du récipient. L'effet technique est de conduire le gaz dès l'entrée vers la sortie.

Avantageusement, le dispositif selon la description comprend un indicateur positionné en aval du second filtre et configuré pour indiquer une concentration d'eau dans le gaz en aval du second filtre au-delà d'une concentration de seuil préalablement définie. L'effet technique est de montrer quand le second filtre est saturé. L'avantage qui en découle est de toujours pouvoir assurer une bonne efficacité d'élimination de l'eau en changeant ou régénérant le matériau adsorbant quand il le faut.

L'invention concerne aussi un ensemble d'analyse en ligne d'un composé cible dans un gaz comprenant un dispositif de nettoyage selon l'invention et un analyseur de gaz en aval du dispositif de nettoyage et configuré pour mesurer la concentration du composé cible dans le gaz. En particulier, l'ensemble d'analyse en ligne d'un composé cible dans un gaz est caractérisé en ce qu'il comprend :
- un dispositif de nettoyage d'un gaz comprenant une pluralité de composés, un premier composé parmi la pluralité de composés étant du goudron, un deuxième composé parmi la pluralité de composés étant de l'eau, et un composé cible, le dispositif de nettoyage comprenant :
   o un récipient avec une entrée et une sortie, le gaz circulant à travers le récipient depuis l'entrée vers la sortie,
   o un premier filtre positionné entre l'entrée et la sortie du récipient, à travers lequel le gaz circule, destiné à filtrer le goudron,
      le premier filtre comprenant un matériau absorbant du goudron,
      le premier filtre ne captant pas ledit composé cible,
      le goudron se trouvant sous forme gazeuse, liquide et/ou solide, et
   o un second filtre positionné entre l'entrée et la sortie du récipient, en amont ou en aval du premier filtre, à travers lequel le gaz circule et destiné à enlever l'eau du gaz
      en ce que le second filtre comprend un matériau adsorbant de l'eau,
      et en ce que le second filtre ne capte pas ledit composé cible ;
- un analyseur de gaz en aval du dispositif de nettoyage et configuré pour mesurer la concentration du composé cible dans le gaz,
et en ce que le composé cible est choisi parmi HCl et NH3.

L'invention concerne également un procédé de filtration sélective d'un gaz en vue de son analyse, le gaz comprenant une pluralité de composés, un premier composé parmi la pluralité de composés étant du goudron, le goudron se trouvant sous forme gazeuse, liquide et/ou solide, un deuxième composé qui est de l'eau et un composé cible choisi parmi HCl et NH3, le procédé comprend les étapes suivantes :
- Circulation du gaz à travers un récipient entre une entrée du récipient et une sortie du récipient,
- Filtration du goudron par un premier filtre positionné entre l'entrée et la sortie du récipient et comprenant un matériau absorbant du goudron ne captant pas ledit composé cible.
- Filtration de l'eau par un second filtre comprenant un matériau adsorbant de l'eau ne captant pas le composé cible et positionné entre l'entrée (14) et la sortie (15) du récipient (13, 93), en amont ou en aval du premier filtre (11, 91).

L'invention sera mieux comprise et d'autres avantages apparaîtront à la lecture de la description détaillée d'un mode de réalisation donné à titre d'exemple, description illustrée par le dessin joint dans lequel :
- la figure 1 représente schématiquement un premier mode de réalisation d'un dispositif de nettoyage avec un premier filtre selon l'invention,
- la figure 2 représente schématiquement un deuxième mode de réalisation d'un dispositif de nettoyage selon l'invention,
- la figure 3 représente schématiquement un autre mode de réalisation d'un dispositif de nettoyage avec un tube à section non-constante selon l'invention,
- la figure 4 représente schématiquement un autre mode de réalisation d'un dispositif de nettoyage selon l'invention,
- la figure 5 représente schématiquement un autre mode de réalisation d'un dispositif de nettoyage selon l'invention,
- la figure 6 représente schématiquement un autre mode de réalisation d'un dispositif de nettoyage avec un second filtre en aval du premier filtre selon l'invention,
- la figure 7 représente schématiquement un autre mode de réalisation d'un dispositif de nettoyage avec un second filtre en amont du premier filtre selon l'invention,
- la figure 8 représente schématiquement un autre mode de réalisation d'un dispositif de nettoyage avec un dispositif de chauffage selon l'invention,
- la figure 9 représente schématiquement un autre mode de réalisation d'un dispositif de nettoyage comprenant plusieurs récipients selon l'invention,
- les figures 10A et 10B représentent le spectre d'infrarouge de 1000ppm d'HCl dans la matrice d'azote pur et le spectre d'infrarouge du gaz contenant le HCl produit par la pyrolyse à 750°C du PVC pur et un mélange de bois et 1% en masse de PVC, échantillonné avec une méthode classique,
- les figures 11A et 11B représentent le spectre d'infrarouge de 1000ppm d'HCl dans la matrice d'azote pur et le spectre d'infrarouge du gaz contenant le HCl produit par la pyrolyse à 750°C du PVC pur et un mélange de bois et 1% en masse de PVC, échantillonné avec le dispositif selon l'invention,

Par souci de clarté, les mêmes éléments porteront les mêmes repères dans les différentes figures.

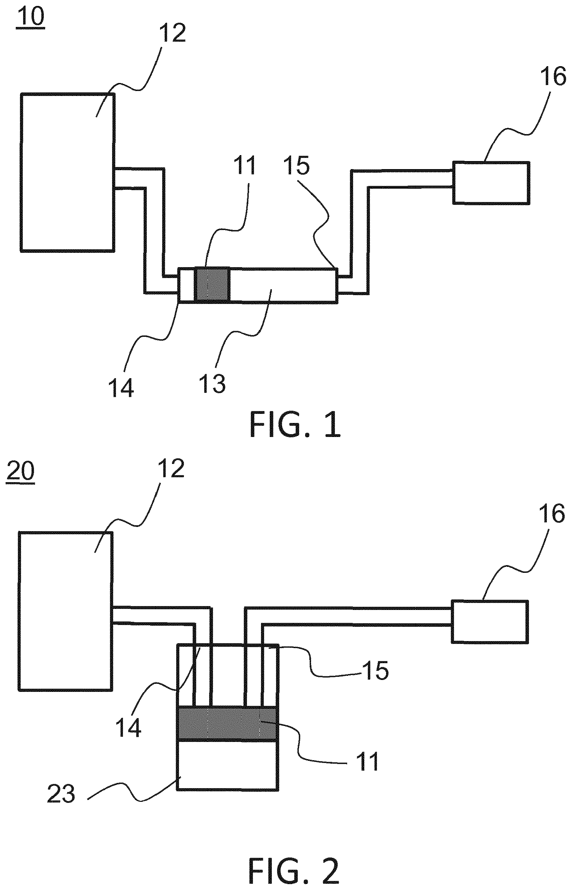

La **figure 1** représente schématiquement un premier mode de réalisation d'un dispositif 10 de nettoyage avec un premier filtre 11 selon l'invention. Le dispositif 10 de nettoyage d'un gaz 12 comprenant une pluralité de composés et un composé cible, un premier composé parmi la pluralité de composés étant du goudron, comprend un récipient 13 avec une entrée 14 et une sortie 15, le gaz 12 circulant à travers le récipient 13 depuis l'entrée 14 vers la sortie 15. Le dispositif 10 comprend le premier filtre 11 positionné entre l'entrée 14 et la sortie 15 du récipient 13, à travers lequel le gaz 12 circule, destiné à filtrer le goudron. Et le premier filtre 11 comprend un matériau absorbant du goudron et le premier filtre 11 ne capte pas ledit composé cible. Le goudron se trouve sous forme gazeuse, liquide et/ou solide. Le gaz 12 comprend une pluralité d'éléments et il est souhaitable d'enlever certains de ces éléments, notamment le goudron, mais d'en conserver d'autres, dont le composé cible, par exemple le HCl ou le H₂S. Le premier filtre 11 comprenant un matériau absorbant du goudron permet d'absorber le goudron, et donc de l'enlever du gaz 12, sans pour autant capter les éléments que l'on souhaite laisser dans le gaz 12.

Le gaz est de type gaz de synthèse. Il peut notamment s'agir du syngaz. Le gaz cible est typiquement un gaz « non organique », notamment un gaz acide tel que HCl ou H₂S, ou un gaz basique tel que NH₃. Le gaz cible est typiquement détecté par spectroscopie optique.

Le goudron, en fonction de la température de l'air à analyser, se trouve non seulement sous forme gazeuse, mais également sous forme liquide et/ou solide. Typiquement, le gaz de synthèse se trouve à une température comprise entre 100°C et 1000°C, notamment entre 100°C et 800°C, de préférence entre 100°c et 400°C. Généralement, le gaz à analyser est à une température de 150°C à l'entrée du dispositif de nettoyage.

En aval du dispositif 10 selon l'invention, il est possible de raccorder un analyseur de gaz 16.

Le récipient 13 peut être en différents matériaux, par exemple en verre ou en quartz.

Le matériau absorbant peut être un matériau naturel, préférentiellement du coton. Alternativement, le matériau absorbant peut être un matériau synthétique, préférentiellement du nylon. Le coton et le nylon sont des matériaux particulièrement absorbants de goudron et il a été constaté qu'ils sont particulièrement indiqués comme matériau absorbant du premier filtre 11.

La **figure 2** représente schématiquement un deuxième mode de réalisation d'un dispositif 20 de nettoyage selon l'invention. Tous les éléments de la figure 2 sont identiques à ceux de la figure 1, excepté le récipient. Sur la figure 2, le récipient est une bouteille 23. L'entrée 14 et la sortie 15 du récipient sont représentées à l'extrémité supérieure du récipient mais pourraient se situer, toutes deux ou une seule des deux, sur une face latérale, au-dessus du premier filtre 11.

Sur la figure 1, le récipient 13 est un tube comprenant deux extrémités, une première parmi les deux extrémités étant l'entrée 14 et une seconde parmi les deux extrémités étant la sortie 15. Le récipient 13 en forme de tube présente l'avantage d'éviter la stagnation de certains éléments du gaz 12 dans le récipient 13. Il n'y a pas de zone morte dans le récipient 13. La vitesse est un paramètre important dans le dispositif selon l'invention pour permettre une bonne analyse en continu du gaz 12.

Sur la figure 1, le tube 13 a une section sensiblement constante entre l'entrée 14 et la sortie 15. La section constante du tube 13 permet d'assurer une vitesse d'écoulement constante du gaz à travers le tube 13. Ainsi, la vitesse du gaz 12 à travers le tube 13 est connue à tout instant.

Le tube peut être de dimensions qui permettent de contrôler le débit d'entrée du gaz à une vitesse cible. Par exemple, ces dimensions peuvent être une longueur de 120 mm et un diamètre interne de 10 mm. Une géométrie fine, c'est-à-dire une relation entre longueur et diamètre de 10 à 15 est avantageuse pour contrôler le débit d'entrée en utilisant des moyens de tirage.

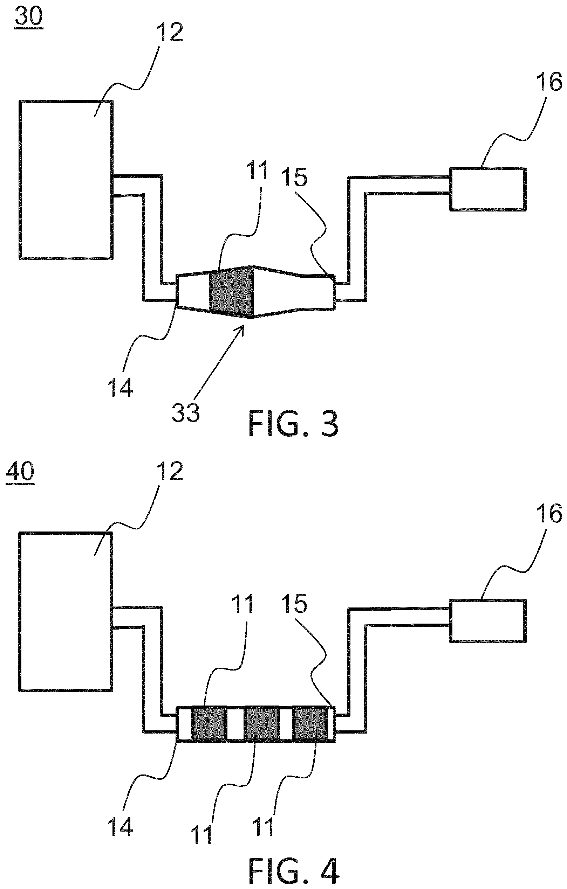

La **figure 3** représente schématiquement un autre mode de réalisation d'un dispositif 30 de nettoyage avec un tube à section non-constante selon l'invention. Tous les éléments de la figure 3 sont identiques à ceux de la figure 1, excepté le récipient référencé ici 33. Dans le mode de réalisation de la figure 3, le récipient 33 est un tube qui a une section non constante entre l'entrée 14 et la sortie 15. Du fait de la section non constante du tube, la vitesse d'écoulement du gaz 12 à travers le tube varie en relation avec la section du tube. Plus la section est grande, plus la vitesse d'écoulement du gaz 12 diminue et plus la section diminue, plus la vitesse d'écoulement du gaz 12 augmente, à la même température et pression. En faisant varier la section du tube, il est possible de placer judicieusement le premier filtre 11 de sorte que la vitesse d'écoulement du gaz 12 à cet endroit du tube soit plus faible. Il en résulte un temps de résidence du gaz à travers le filtre plus long et donc un filtrage accru des éléments souhaités tels que le goudron.

Sur la figure 3, une seule variation de section du tube 33 est représentée mais l'invention s'applique de façon similaire à plusieurs variations successives de section du tube 33, croissantes et/ou décroissantes.

La **figure 4** représente schématiquement un autre mode de réalisation d'un dispositif 40 de nettoyage selon l'invention. Tous les éléments de la figure 4 sont identiques à ceux de la figure 1. Le dispositif 40 comprend plusieurs premiers filtres 11 disposés successivement dans le tube. Le fait d'ajouter plusieurs premiers filtres 11 permet d'obtenir un meilleur filtrage des goudrons dans le gaz 12. En effet, les goudrons non filtrés par le premier des premiers filtres 11 doivent traverser le deuxième, et éventuellement le troisième des premiers filtres 11.

Sur la figure 4, le dispositif 40 de nettoyage est représenté avec un tube mais le récipient pourrait tout aussi bien être une bouteille.

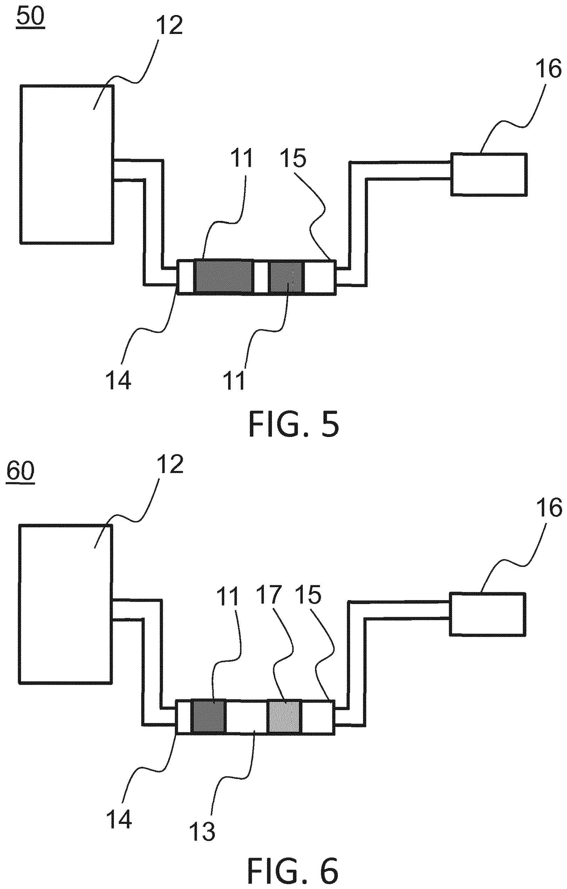

La **figure 5** représente schématiquement un autre mode de réalisation d'un dispositif 50 de nettoyage selon l'invention. Tous les éléments de la figure 5 sont identiques à ceux de la figure 1. Le dispositif 50 comprend plusieurs premiers filtres 11 disposés successivement dans le tube, par exemple ici deux, et l'un des premiers filtres est plus long que l'autre. Autrement dit, le gaz 12 a un temps de passage plus long à travers le premier filtre. Outre la meilleure filtration des goudrons dans le gaz 12 obtenue par la mise en place de plusieurs premiers filtres, le fait d'avoir un premier filtre 11 plus long selon l'axe selon lequel le récipient s'étend permet encore d'améliorer la capacité d'élimination des goudrons.

La **figure 6** représente schématiquement un autre mode de réalisation d'un dispositif 60 de nettoyage avec un second filtre 17 en aval du premier filtre 11 selon l'invention. Un deuxième composé parmi plusieurs composés à éliminer dans le gaz 12 est de l'eau. Le dispositif 60 de nettoyage selon l'invention comprend le second filtre 17 positionné entre l'entrée 14 et la sortie 15 du récipient 13, en amont ou en aval du premier filtre 11, à travers lequel le gaz 12 circule et destiné à enlever l'eau du gaz 12. Le second filtre 17 comprend un matériau adsorbant de l'eau.

La juxtaposition d'un premier filtre 11 et d'un second filtre 17 dans le récipient 13 permet d'avoir à la fois un matériau absorbant de goudron et un matériau adsorbant d'eau, ce qui permet à la fois, dans un même récipient 13, d'enlever les goudrons et l'eau du gaz 12.

Sur la figure 6, et comme c'est le cas pour chaque mode de réalisation représenté dans cette demande, le dispositif 60 de nettoyage est représenté avec un tube mais le récipient pourrait tout aussi bien être une bouteille.

Enfin, le nombre de premiers filtres 11 et de seconds filtres 17 peut varier, de un premier filtre 11 à plusieurs premiers filtres 11 et/ou de un second filtre 17 à plusieurs seconds filtres 17.

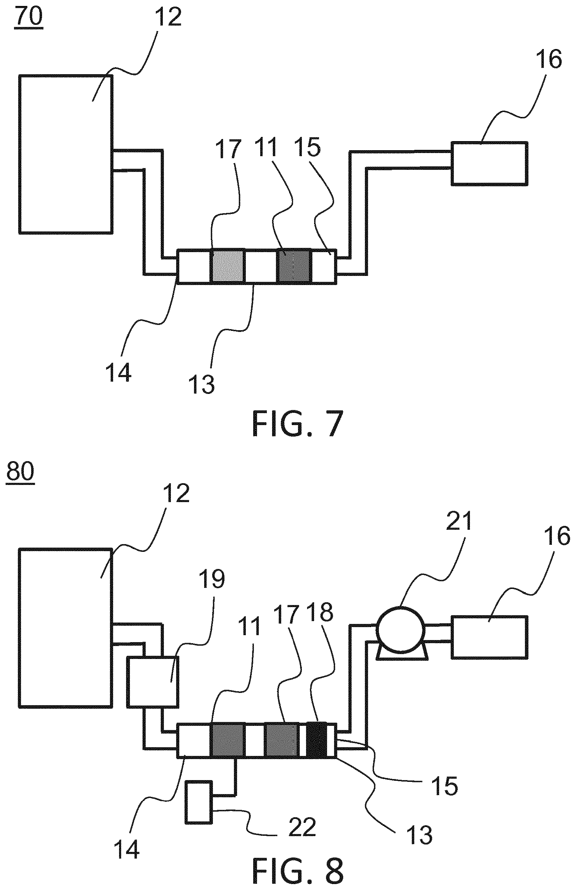

La **figure 7** représente schématiquement un autre mode de réalisation d'un dispositif 70 de nettoyage avec un second filtre 17 en amont du premier filtre 11 selon l'invention. Tous les éléments de la figure 7 sont identiques à ceux de la figure 6. La seule différence se situe dans le positionnement du second filtre 17 qui est cette fois positionné en amont du premier filtre 11 dans le récipient 13. L'ordre dans lequel les premier et second filtres sont positionnés dans le récipient n'a pas d'impact sur leur capacité filtrante.

Dans le cas où un troisième composé parmi la pluralité de composés dans le gaz 12 est un gaz acide tel que HCl, le matériau adsorbant de l'eau comprend une surface de particule neutre et/ou acide. Le pH de surface du matériau adsorbant est donc inférieur ou égal à 7.0. La surface de particule neutre ou acide du matériau adsorbant de l'eau empêche toute interaction chimique ou physique du composé à analyser, tel que HCl, avec la surface du matériau.

Dans le cas où le troisième composé à analyser dans le gaz 12 est un gaz basique, tel que NH₃, le matériau adsorbant de l'eau comprend une surface de particule neutre et/ou basique. Le pH de surface du matériau adsorbant est donc supérieur ou égal à 7.0. La surface de particule neutre ou basique du matériau adsorbant de l'eau empêche toute interaction chimique ou physique du composé à analyser, tel que NH₃, avec la surface du matériau.

Le matériau adsorbant peut être une zéolithe. Le pH de surface d'une zéolithe peut être contrôlé par la mise en contact avec une solution acide ou basique. Une zéolithe peut avoir un seul type de porosité signifiant la bonne homogénéité de la taille des pores. En choisissant judicieusement la zéolithe utilisée dans le second filtre 17, la zéolithe adsorbe l'eau et laisse passer le ou les éléments souhaités comme le HCl.

La **figure 8** représente schématiquement un autre mode de réalisation d'un dispositif 80 de nettoyage avec un dispositif de chauffage selon l'invention. Tous les éléments de la figure 8 sont identiques à ceux de la figure 6. Dans ce mode de réalisation, le dispositif 80 de nettoyage comprend un dispositif de chauffage 19 du gaz 12 positionné en amont de l'entrée 14 du récipient 13. Avantageusement, le chauffage du gaz est effectué en amont du récipient et le second filtre 17 est également chauffé.

La température du gaz 12 et du second filtre 17 est importante. En effet, l'eau ne doit pas condenser. Le dispositif de chauffage 19 permet de chauffer le gaz 12 et le second filtre 17 de sorte à maintenir la température du second filtre 17 au-dessus du point de rosée de l'eau, ce qui évite le phénomène de condensation de l'eau.

En outre, le dispositif selon l'invention peut comprendre un dispositif de refroidissement 22 du premier filtre 11. Un premier effet technique issu de l'utilisation du dispositif de refroidissement 22 du premier filtre 11 est d'augmenter la condensation des goudrons et donc l'élimination des goudrons plus efficace avant que le gaz 12 ne sorte par la sortie 15. Il y a augmentation de l'efficacité de purification. Un deuxième effet technique issu de l'utilisation du dispositif de refroidissement 22 du premier filtre 11 est que, en combinaison avec des matériaux synthétiques, une baisse de température empêche certains composés comme le HCl contenu dans le gaz 12 de réagir avec les matériaux.

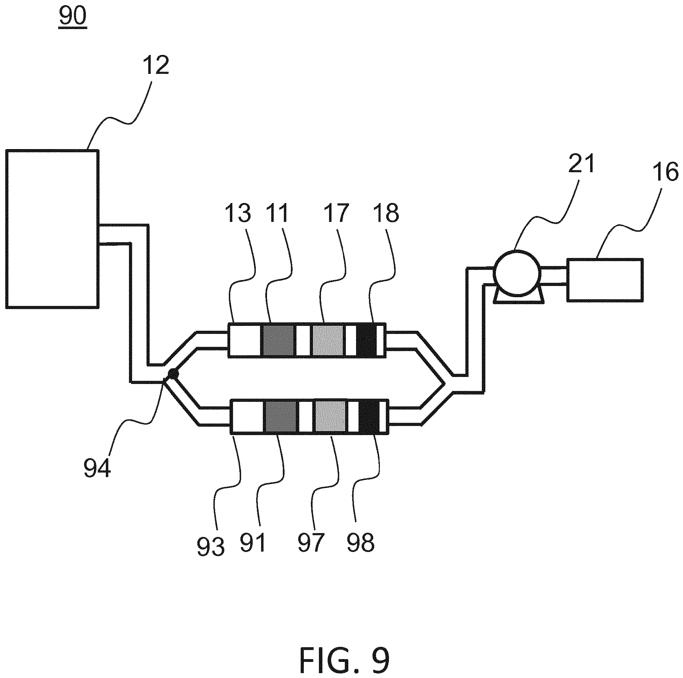

A noter que le dispositif de refroidissement 22 du premier filtre 11 est représenté de façon très schématique sur la figure 8. Ce dispositif de refroidissement est avantageux mais pas obligatoire. Il n'apparaît pas sur les autres figures mais est bien entendu combinable avec les éléments présentés aux figures 1 à 7 ainsi qu'à la figure 9.

Le dispositif selon l'invention peut aussi comprendre un dispositif d'aspiration 21 du gaz 12 positionné en aval de la sortie 15 du récipient 13. Le disposition d'aspiration 21 peut être par exemple une pompe, et permet de conduire le gaz 12 dès l'entrée 14 vers la sortie 15.

A noter que la même remarque faite pour le dispositif de refroidissement 22 du premier filtre 11 s'applique au dispositif d'aspiration 21 : il est représenté de façon très schématique sur la figure 8. Ce dispositif est avantageux mais pas obligatoire. Il n'apparaît pas sur les autres figures mais est bien entendu combinable avec les éléments présentés aux figures 1 à 7 ainsi qu'à la figure 9.

Le dispositif selon l'invention peut comprendre un indicateur 18 positionné en aval du second filtre 17 et configuré pour indiquer une concentration d'eau dans le gaz 12 en aval du second filtre 17 au-delà d'une concentration de seuil préalablement définie. Il peut s'agir par exemple d'un gel de silice qui change de couleur au-delà d'une certaine concentration en eau. Cet indicateur 18 permet de montrer que le second filtre 17 n'est plus assez efficace pour l'élimination. Le matériau adsorbant du second filtre 17 adsorbe l'eau, et au-delà d'une certaine utilisation, il faut le changer ou le régénérer.

La **figure 9** représente schématiquement un autre mode de réalisation d'un dispositif 90 de nettoyage comprenant plusieurs récipients selon l'invention. Tous les éléments de la figure 9 sont identiques à ceux de la figure 8. Le dispositif 90 comprend en outre un deuxième récipient 93, en parallèle du récipient 13, dans lequel se trouve un premier filtre 91 et un second filtre 97 (en amont ou en aval du premier filtre 91, sur la figure 9 il est en aval) ainsi qu'un indicateur 98. Le dispositif 90 comprend un doigt de dérivation 94 en amont des récipients 13, 93. Sur la figure 9, le doigt de dérivation 94 est positionné vers le récipient 93, c'est-à-dire qu'il empêche le gaz 12 de circuler à travers le récipient 93. Tout le gaz 12 est donc dirigé vers le récipient 13 qu'il traverse pour être nettoyé.

Au bout d'une certaine durée d'utilisation, l'indicateur 18 du récipient 13 indique une concentration d'eau dans le gaz 12 en aval du second filtre 17 au-delà d'une concentration de seuil préalablement définie. Il faut alors changer/régénérer le matériau adsorbant du second filtre 17. Le doigt de dérivation 94 est alors basculé vers le récipient 13, c'est-à-dire qu'il empêche le gaz 12 de circuler à travers le récipient 13. Tout le gaz 12 est alors dirigé vers le récipient 93 qu'il traverse pour être nettoyé. La portion du récipient 13 étant mise hors de service, il est alors aisé de changer et/ou régénérer le matériau adsorbant du second filtre 17. En même temps, il est aussi possible de changer le matériau absorbant du premier filtre 11 pour garantir une meilleure efficacité de filtrationlors de l'utilisation suivante du récipient 13.

Quand l'indicateur 98 du récipient 93 indique une concentration d'eau dans le gaz 12 en aval du second filtre 17 au-delà d'une concentration de seuil préalablement définie, le doigt de dérivation 94 est alors basculé vers le récipient 93, c'est-à-dire qu'il empêche le gaz 12 de circuler à travers le récipient 93. Et il est alors possible d'effectuer la même manoeuvre que précédemment pour les filtres du récipient 13.

A noter qu'il est aussi possible de positionner le doigt de dérivation 94 en position neutre, c'est-à-dire en position sensiblement horizontale sur la figure 9, de sorte à permettre l'écoulement du gaz 12 dans les deux récipients 13 et 93. Cela permet un plus grand débit d'entrée du gaz 12, et donc une plus grande capacité de nettoyage de gaz.

Ce principe est aussi applicable à plusieurs récipients.

Le basculement du doigt de dérivation 94 peut également être piloté dans une boucle de commande avec détection quand l'indicateur 18 d'un récipient indique une concentration d'eau dans le gaz 12 en aval du second filtre 17 au-delà d'une concentration de seuil préalablement définie et basculement du doigt de dérivation 94 vers ledit récipient pour diriger le gaz vers un autre récipient.

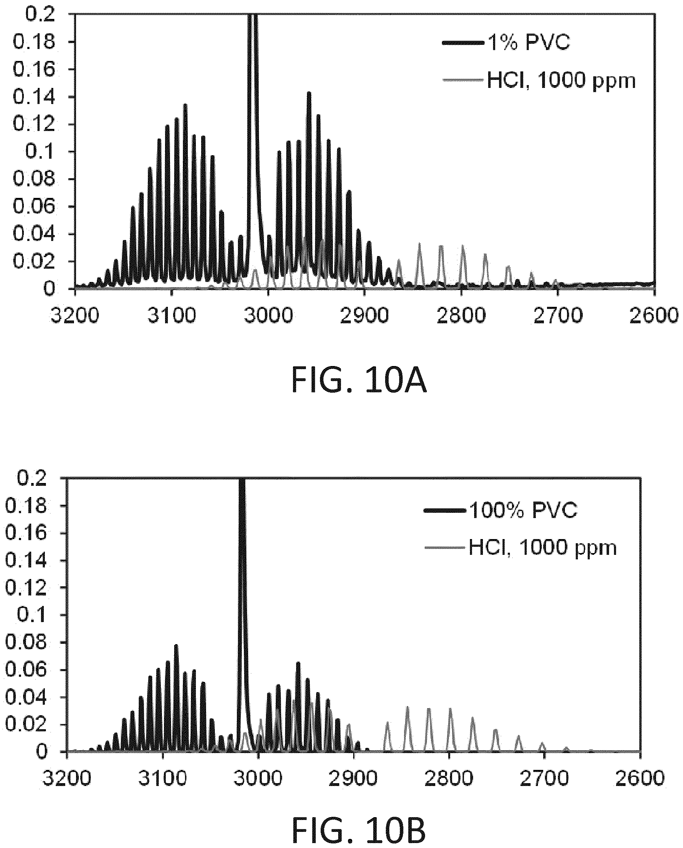

Les **figures 10A et 10B** représentent le spectre d'infrarouge contenant 1000ppm d'HCl dans la matrice d'azote pur et le spectre infrarouge du gaz contenant le HCl produit par la pyrolyse à 750°C du PVC pur et un mélange de bois et 1% en masse de PVC, échantillonné avec une méthode classique, en l'occurrence en utilisant le solvant isopropanol. Sur ces figures, l'absorbance est tracée est fonction de la longueur d'onde. On peut voir sur les figures 10A et 10B qu'il n'y a pas de pics d'absorption qui correspondent à HCl et donc pas de détection du HCl. On peut en conclure par conséquent que la méthode classique d'échantillonnage du HCl dans le syngas n'est pas adéquate.

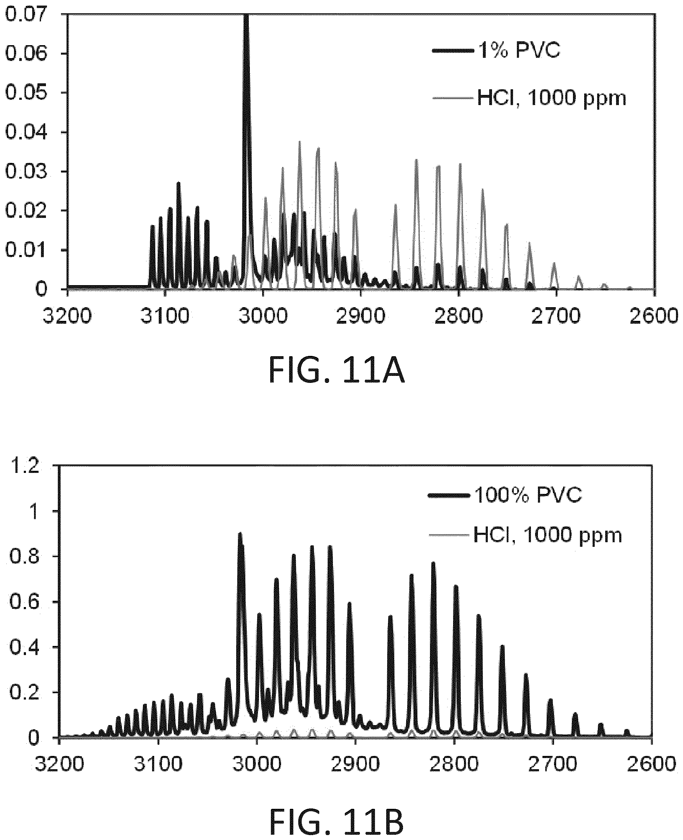

Les **figures 11A et 11B** représentent le spectre d'infrarouge de 1000ppm d'HCl dans la matrice d'azote pur et le spectre d'infrarouge du gaz contenant le HCl produit par la pyrolyse à 750°C du PVC pur et un mélange de bois et 1% en masse de PVC, échantillonné avec le dispositif selon l'invention. Sur ces figures, l'absorbance est tracée est fonction de la longueur d'onde. La figure 11A montre qu'en utilisant le dispositif selon l'invention pour échantillonner du HCl dans un gaz produit par la pyrolyse du PVC pur, on peut observer les pics d'absorbance qui correspondent aux pics du HCl dans la région 2600 - 2900 cm⁻¹ du spectre. La même observation peut être faite sur la figure 11B pour le gaz produit par la pyrolyse d'un mélange du bois et 1% en masse de PVC. Cette fois-ci, la hauteur des pics d'absorbance est plus petite en raison de la concentration plus faible du HCl dans le syngas. On peut donc conclure que le dispositif selon l'invention permet d'échantillonner un gaz contenant même une faible concentration initiale de HCl.

Le procédé de filtration sélective d'un gaz 12 selon l'invention comprenant une pluralité de composés, un premier composé parmi la pluralité de composés étant du goudron, le goudron se trouvant sous forme gazeuse, liquide et/ou solide et un composé cible, comprend une étape de circulation du gaz 12 à travers un récipient 13 entre une entrée 14 du récipient et une sortie 15 du récipient. Le procédé comprend une étape de filtration du goudron par un premier filtre 11 positionné entre l'entrée 14 et la sortie 15 du récipient 13 et comprenant un matériau absorbant du goudron ne captant pas ledit composé cible.

Dans le cas où le gaz 12 comprend un deuxième composé qui est de l'eau, le récipient 13 peut comprendre un second filtre comprenant un matériau adsorbant de l'eau ne captant pas le composé cible et positionné entre l'entrée 14 et la sortie 15 du récipient, en amont ou en aval du premier filtre. Le gaz 12 circule à travers le récipient, et le second filtre est destiné à enlever l'eau di gaz 12. Le procédé de filtration comprend alors une étape de filtration de l'eau par le second filtre.

## Revendications

1. Ensemble d'analyse en ligne d'un composé cible dans un gaz **caractérisé en ce qu'**il comprend :
• un dispositif de nettoyage (10, 20, 30, 40, 50, 60, 70, 80, 90) d'un gaz (12) comprenant une pluralité de composés, un premier composé parmi la pluralité de composés étant du goudron, un deuxième composé parmi la pluralité de composés étant de l'eau, et un composé cible, le dispositif de nettoyage comprenant :
o un récipient (13, 93) avec une entrée (14) et une sortie (15), le gaz (12) circulant à travers le récipient (13, 93) depuis l'entrée (14) vers la sortie (15),
o un premier filtre (11, 91) positionné entre l'entrée (14) et la sortie (15) du récipient (13, 93), à travers lequel le gaz (12) circule, destiné à filtrer le goudron,
le premier filtre (11, 91) comprenant un matériau absorbant du goudron,
le premier filtre (11, 91) ne captant pas ledit composé cible,
le goudron se trouvant sous forme gazeuse, liquide et/ou solide, et
o un second filtre (17, 97) positionné entre l'entrée (14) et la sortie (15) du récipient (13, 93), en amont ou en aval du premier filtre (11, 91), à travers lequel le gaz (12) circule et destiné à enlever l'eau du gaz (12)
**en ce que** le second filtre comprend un matériau adsorbant de l'eau,
et **en ce que** le second filtre ne capte pas ledit composé cible ;
• un analyseur de gaz (16) en aval du dispositif de nettoyage et configuré pour mesurer la concentration du composé cible dans le gaz,
et **en ce que** le composé cible est choisi parmi HCl et NH3.

2. Ensemble d'analyse en ligne selon la revendication 1, **caractérisé en ce que** le matériau absorbant est un matériau naturel, préférentiellement du coton.

3. Ensemble d'analyse en ligne selon la revendication 1, **caractérisé en ce que** le matériau absorbant est un matériau synthétique, préférentiellement du nylon.

4. Ensemble d'analyse en ligne selon l'une quelconque des revendications précédentes, un troisième composé parmi la pluralité de composés étant un gaz acide, **caractérisé en ce que** le matériau adsorbant de l'eau comprend une surface de particule neutre ou acide.

5. Ensemble d'analyse en ligne selon l'une quelconque des revendications précédentes, **caractérisé en ce que** le matériau adsorbant est une zéolithe.

6. Ensemble d'analyse en ligne selon l'une quelconque des revendications précédentes, **caractérisé en ce que** le récipient (13, 93) est un tube comprenant deux extrémités, une première parmi les deux extrémités étant l'entrée et une seconde parmi les deux extrémités étant la sortie.

7. Ensemble d'analyse en ligne selon la revendication 6, **caractérisé en ce que** le tube a une section sensiblement constante entre l'entrée et la sortie.

8. Ensemble d'analyse en ligne selon la revendication 6, **caractérisé en ce que** le tube a une section non constante entre l'entrée et la sortie.

9. Ensemble d'analyse en ligne selon l'une quelconque des revendications 4 à 8, **caractérisé en ce qu'**il comprend un dispositif de chauffage (19) du gaz positionné en amont de l'entrée (14) du récipient (13, 93).

10. Ensemble d'analyse en ligne selon l'une quelconque des revendications précédentes, **caractérisé en ce qu'**il comprend un dispositif de refroidissement (22) du premier filtre (11).

11. Ensemble d'analyse en ligne selon l'une quelconque des revendications précédentes, **caractérisé en ce qu'**il comprend un dispositif d'aspiration (21) du gaz (12) positionné en aval de la sortie (15) du récipient (13, 93).

12. Ensemble d'analyse en ligne selon l'une quelconque des revendications 4 à 11, **caractérisé en ce qu'**il comprend un indicateur (18, 98) positionné en aval du second filtre (17, 97) et configuré pour indiquer une concentration d'eau dans le gaz (12) en aval du second filtre (17, 97) au-delà d'une concentration de seuil préalablement définie.

13. Procédé de filtration sélective d'un gaz (12) en vue de son analyse, le gaz comprenant une pluralité de composés, un premier composé parmi la pluralité de composés étant du goudron, le goudron se trouvant sous forme gazeuse, liquide et/ou solide, un deuxième composé qui est de l'eau et un composé cible choisi parmi HClet NH3, **caractérisé en ce qu'**il comprend les étapes suivantes :
• Circulation du gaz (12) à travers un récipient (13, 93) entre une entrée (14) du récipient et une sortie (15) du récipient,
• Filtration du goudron par un premier filtre (11, 91) positionné entre l'entrée (14) et la sortie (15) du récipient (13, 93) et comprenant un matériau absorbant du goudron ne captant pas ledit composé cible,
• Filtration de l'eau par un second filtre comprenant un matériau adsorbant de l'eau ne captant pas le composé cible et positionné entre l'entrée (14) et la sortie (15) du récipient (13, 93), en amont ou en aval du premier filtre (11, 91).

## Patentansprüche

1. Einheit zur Online-Analyse einer Zielverbindung in einem Gas, **dadurch gekennzeichnet, dass** sie umfasst:
• eine Reinigungsvorrichtung (10, 20, 30, 40, 50, 60, 70, 80, 90) eines Gases (12), eine Vielzahl von Verbindungen umfassend, wobei eine erste Verbindung aus der Vielzahl von Verbindungen Teer ist, eine zweite Verbindung aus der Vielzahl von Verbindungen Wasser ist, und eine Zielverbindung, wobei die Reinigungsvorrichtung umfasst:
∘ einen Behälter (13, 93) mit einem Einlass (14) und einem Auslass (15), wobei das Gas (12) durch den Behälter (13, 93) vom Einlass (14) zum Auslass (15) strömt,
∘ einen ersten Filter (11, 91), der zwischen dem Einlass (14) und dem Auslass (15) des Behälters (13, 93), durch den das Gas (12) strömt, angeordnet ist, und dazu bestimmt ist, den Teer zu filtern,
wobei der erste Filter (11, 91) ein Material umfasst, das den Teer absorbiert,
wobei der erste Filter (11, 91) die Zielverbindung nicht auffängt,
wobei der Teer in gasförmiger, flüssiger und/oder fester Form vorliegt, und
o einen zweiten Filter (17, 97), der zwischen dem Einlass (14) und dem Auslass (15) des Behälters (13, 93), durch den das Gas (12) strömt, stromaufwärtig oder stromabwärtig des ersten Filters (11, 91), angeordnet ist, und dazu bestimmt ist, das Wasser aus dem Gas (12) zu entfernen,
dadurch, dass der zweite Filter ein Material umfasst, das Wasser adsorbiert,
und dass der zweite Filter die Zielverbindung nicht auffängt;
• einen Gasanalysator (16), stromabwärtig der Reinigungsvorrichtung, und konfiguriert, um die Konzentration der Zielverbindung im Gas zu messen,
und dass die Zielverbindung aus HCl und NH3 ausgewählt wird.

2. Einheit zur Online-Analyse nach Anspruch 1, **dadurch gekennzeichnet, dass** das absorbierende Material ein natürliches Material ist, vorzugsweise Baumwolle.

3. Einheit zur Online-Analyse nach Anspruch 1, **dadurch gekennzeichnet, dass** das absorbierende Material ein synthetisches Material ist, vorzugsweise Nylon.

4. Einheit zur Online-Analyse nach einem der vorhergehenden Ansprüche, wobei eine dritte Verbindung aus der Vielzahl von Verbindungen ein saures Gas ist, **dadurch gekennzeichnet, dass** das Material, das Wasser adsorbiert, eine neutrale oder saure Partikeloberfläche umfasst.

5. Einheit zur Online-Analyse nach einem der vorhergehenden Ansprüche, **dadurch gekennzeichnet, dass** das adsorbierende Material ein Zeolith ist.

6. Einheit zur Online-Analyse nach einem der vorhergehenden Ansprüche, **dadurch gekennzeichnet, dass** der Behälter (13, 93) ein Rohr ist, das zwei Enden umfasst, wobei ein erstes von den zwei Enden der Einlass ist und ein zweites von den zwei Enden der Auslass ist.

7. Einheit zur Online-Analyse nach Anspruch 6, **dadurch gekennzeichnet, dass** das Rohr einen im Wesentlichen konstanten Querschnitt zwischen dem Einlass und dem Auslass aufweist.

8. Einheit zur Online-Analyse nach Anspruch 6, **dadurch gekennzeichnet, dass** das Rohr einen nicht konstanten Querschnitt zwischen dem Einlass und dem Auslass aufweist.

9. Einheit zur Online-Analyse nach einem der Ansprüche 4 bis 8, **dadurch gekennzeichnet, dass** sie eine Vorrichtung zum Wärmen (19) des Gases umfasst, die stromaufwärtig des Einlasses (14) des Behälters (13, 93) angeordnet ist.

10. Einheit zur Online-Analyse nach einem der vorhergehenden Ansprüche, **dadurch gekennzeichnet, dass** sie eine Vorrichtung zur Kühlung (22) des ersten Filters (11) umfasst.

11. Einheit zur Online-Analyse nach einem der vorhergehenden Ansprüche, **dadurch gekennzeichnet, dass** sie eine Vorrichtung zum Ansaugen (21) des Gases (12) umfasst, die stromabwärtig des Auslasses (15) des Behälters (13, 93) angeordnet ist.

12. Einheit zur Online-Analyse nach einem der Ansprüche 4 bis 11, **dadurch gekennzeichnet, dass** sie einen Indikator (18, 98) umfasst, der stromabwärtig des zweiten Filters (17, 97) angeordnet ist und konfiguriert ist, um eine Wasserkonzentration im Gas (12) stromabwärtig des zweiten Filters (17, 97) anzuzeigen, die über eine zuvor definierte Konzentrationsschwelle hinausgeht.

13. Verfahren zum selektiven Filtern eines Gases (12) im Hinblick auf seine Analyse, wobei das Gas eine Vielzahl von Verbindungen umfasst, eine erste Verbindung aus der Vielzahl von Verbindungen, die Teer ist und der Teer in gasförmiger, flüssiger und/oder fester Form vorliegt, eine zweite Verbindung, die Wasser ist, und eine Zielverbindung, die aus HCl und NH3 ausgewählt wird, **dadurch gekennzeichnet, dass** es die folgenden Schritte umfasst:
• Strömung eines Gases (12) durch einen Behälter (13, 93) zwischen einem Einlass (14) des Behälters und einem Auslass (15) des Behälters,
• Filterung des Teers durch einen ersten Filter (11, 91), der zwischen dem Einlass (14) und dem Auslass (15) des Behälters (13, 93) angeordnet ist, und ein Material umfasst, das Teer absorbiert und die Zielverbindung nicht auffängt,
• Filterung des Wassers durch einen zweiten Filter, der ein Material umfasst, das Wasser adsorbiert und die Zielverbindung nicht auffängt, und zwischen dem Einlass (14) und dem Auslass (15) des Behälters (13, 93), stromaufwärtig oder stromabwärtig des ersten Filters (11, 91) angeordnet ist.

## Claims

1. Assembly for the online analysis of a target compound in a gas, **characterised in that** it comprises:
• a device (10, 20, 30, 40, 50, 60, 70, 80, 90) for cleaning a gas (12) comprising a plurality of compounds, a first compound from the plurality of compounds being tar, a second compound from the plurality of compounds being water, and a target compound, the cleaning device comprising:
∘ a receptacle (13, 93) with an inlet (14) and an outlet (15), the gas (12) circulating through the receptacle (13, 93) from the inlet (14) to the outlet (15),
∘ a first filter (11, 91) positioned between the inlet (14) and the outlet (15) of the receptacle (13, 93), through which the gas (12) circulates, intended to filter the tar,
the first filter (11, 91) comprising a material absorbing tar,
the first filter (11, 91) not capturing said target compound,
the tar being in gaseous, liquid and/or solid form, and
o a second filter (17, 97) positioned between the inlet (14) and the outlet (15) of the receptacle (13, 93), upstream or downstream of the first filter (11, 91), through which the gas (12) circulates and intended to remove the water from the gas (12)
**in that** the second filter comprises a material adsorbing water,
and **in that** the second filter does not capture said target compound;
• a gas analyser (16) downstream of the cleaning device and configured to measure the concentration of the target compound in the gas,
and **in that** the target compound is selected from HCl and NH₃.

2. Online analysis assembly according to claim 1, **characterised in that** the absorbent material is a natural material, preferentially cotton.

3. Online analysis assembly according to claim 1, **characterised in that** the absorbent material is a synthetic material, preferentially nylon.

4. Online analysis assembly according to any one of the preceding claims, a third compound from the plurality of compounds being an acid gas, **characterised in that** the water-adsorbent material comprises a neutral or acid particle surface.

5. Online analysis assembly according to any one of the preceding claims, **characterised in that** the adsorbent material is a zeolite.

6. Online analysis assembly according to any one of the preceding claims, **characterised in that** the receptacle (13, 93) is a tube comprising two ends, a first of the two ends being the inlet and the second of the two ends being the outlet.

7. Online analysis assembly according to claim 6, **characterised in that** the tube has a substantially constant cross-section between the inlet and the outlet.

8. Online analysis assembly according to claim 6, **characterised in that** the tube has a non-constant cross-section between inlet and the outlet.

9. Online analysis assembly according to any one of claims 4 to 8, **characterised in that** it comprises a device (19) for heating the gas positioned upstream of the inlet (14) of the receptacle (13, 93).

10. Online analysis assembly according to any one of the preceding claims, **characterised in that** it comprises a device (22) for cooling the first filter (11) .

11. Online analysis assembly according to any one of the preceding claims, **characterised in that** it comprises a device (21) for sucking the gas (12) positioned downstream of the outlet (15) of the receptacle (13, 93).

12. Online analysis assembly according to any one of claims 4 to 11, **characterised in that** it comprises an indicator (18, 98) positioned downstream of the second filter (17, 97) and configured to indicate a concentration of water in the gas (12) downstream of the second filter (17, 97) beyond a previously defined threshold concentration.

13. Method for the selective filtration of a gas (12) with a view to analysis thereof, the gas comprising a plurality of compounds, a first compound from the plurality of compounds being tar, the tar being in gaseous, liquid and/or solid form, a second compound that is water and a target compound selected from HCl and NH₃, **characterised in that** it comprises the following steps:
• Circulating the gas (12) through a receptacle (13, 93) between an inlet (14) of the receptacle and an outlet (15) of the receptacle,
• Filtering the tar through a first filter (11, 91) positioned between the inlet (14) and the outlet (15) of the receptacle (13, 93) and comprising a tar-absorbent material not capturing said target compound,
• Filtering the water through a second filter comprising a water-adsorbent material not capturing the target compound and positioned between the inlet (14) and the outlet (15) of the receptacle (13, 93), upstream or downstream of the first filter (11, 91).
